# EUROPEAN PATENT SPECIFICATION

(11) **EP 1 728 503 B1**
(45) Date of publication and mention of the grant of the patent: **17.09.2008**
(21) Application number: 06011333.9
(22) Date of filing: 01.06.2006
(51) Int. Cl.: A61K 8/97, A61Q 5/00, A61Q 5/12

(54) **Cosmetic hair conditioning composition**
Haarkonditionierende kosmetische Zusammensetzung
Composition cosmétique pour pour le conditionnement des cheveux

(30) Priority: 02.06.2005 EP 05011899
(43) Date of publication of application: 06.12.2006
(73) Proprietor: KPSS-Kao Professional Salon Services GmbH, 64297 Darmstadt (DE)
(72) Inventor: Molenda, Michael, 60598 Frankfurt (DE); Schupp, Bettina, 64404 Bickenbach (DE)

(56) References cited:
- WO-A-20/04017934
- DE-A1- 3 207 004
- US-A1- 2002 098 253
- US-B1- 6 468 564

## Description

The present invention is related to cosmetic composition comprising extracts from Blue Lotus (Nymphaea caerulea) flowers.

Hair conditioning compositions have been known for many decades. Although the state of the art have been quite well established, there is still need for improvements. Natural ingredients have always been attractive to consumer. Especially cosmetic products comprising extracts and/or parts of natural resources have always been perceived as safer than those of purely on synthetic raw materials. Therefore, the efforts have been made to design cosmetic products based on, where possible, purely on natural ingredients.

Natural ingredients derived from Nymphaea species have also found their application in cosmetic products, The use of an extract prepared from seeds of Blue Lotus is disclosed for skin care preparations in US 6,648,564 B1. Skin care application of another species from Nymphaea, Nymphaea alba, is disclosed in a recent PCT publication WO 2004/017934. Furthermore, in 2 Japanese patent application publications JP 2003002813 A and JP 2003002820 A, skin care benefits of Nymphaea species are disclosed. DE 101 39 612 C1 and DE 32 07 004 A1 deal with natural ingredients derived from, among others, Nymphaea species in cosmetic compositions especially designed for skin.

US 2002/0098253 A1 discloses oral compositions comprising Blue Lotus.

As clear from above summary, the prior art in the field of the use of Nymphaea species in the cosmetic field is well enhanced only in preparations designed for skin. In those publications nothing is said on the effects of extracts of Nymphaea species on hair.

Known problems in hair conditioning field are combability, manageability, elasticity, volume and body, and shine of hair after use of products. In various publications effects of cosmetic preparations on these hair properties have been disclosed.

The inventors of the present application have surprisingly discovered that addition of extracts of Blue lotus flower into hair conditioning compositions improves hair shine dramatically. The improvement in combability, elasticity, manageability and especially volume and body has also been observed.

Thus, the object of the present invention is cosmetic composition, especially for hair, comprising extract of Blue Lotus flowers.

Further object of the present invention is conditioning compositions for hair comprising at least one hair conditioning ingredient and extract prepared from flowers of Blue Lotus.

Still further object of the present invention is the use of extract prepared from Blue Lotus flowers for enhancing hair shine, volume and body.

The extracts of Blue lotus flowers is prepared by mixing the blue lotus flowers with propylene glycol and water mixture and incubating the mixture preferably at elevated temperature such as between 40°C and 80°C for an appropriate period of time and removing the undissolved part by filtration. Although mixture of propylene glycol and water at any ratio is appropriate for the preparation of extracts, the preferred solvent composition comprises at least 50%, preferably at least 60%, more preferably at least 70% and most preferably at least 80% by weight propylene glycol, calculated to total solvent composition. The Blue Lotus flower extract comprises approximately 10% by weight of Blue lotus flowers and approximately 10% by weight water, both calculated to total composition of the extract.

The preferred extract of the blue lotus flowers comprises 80% propylene glycol, 10% blue lotus flowers and 10 % water, all values are by weight and calculated to total composition. The extract thus obtained has a density at 20°C between 1.045 and 1.055 g/cm3, refractive index at 20°C between 1.423 and 1.433 and pH value between 5.5 and 7.0. The dry matter of the extract is approximately 10% by weight of the whole extract. Such extracts are commercially available form the company Croda under the trade name Crodarom.

The compositions of the present invention comprises the extract at a concentration of 0.01 % to 3%, preferably 0.01 % to 2.5%, more preferably 0.02% to 2% and most preferably 0.05 to 1% by weight calculated to total composition and as dry matter of the extract.

Compositions for hair according to the present invention are any of those well-known types such as shampoo, conditioner, treatment, lotions, foams, and gels. Those compositions used especially after shampooing can be applied to hair either as a leave-in or as a rinse off compositions.

Compositions of the present invention in the form of hair cleansing preparation can be in the form of conventional liquid thickened shampoo, as well in the form of ready to use foam delivered either from a pump-foamer or from an aerosol bottle. In the, case that an aerosol foam preparation is preferred, propellant gas must be added to the formulation.

Cleansing shampoo composition of the present invention comprises at least one surfactant selected from anionic, non-ionic and/or amphoteric or zwitterionic surfactants at a concentration range of 2 to 40%, preferably 5 to 30% and more preferably 5 to 20% by weight, calculated to the total composition.

Anionic surfactants suitable within the scope of the invention are preferably present in an amount from 1 to about 30%, preferably 2 to 25% and most preferably 2 - 20% by weight, calculated to the total composition.

These are anionic surfactants of the sulfate, sulfonate, carboxylate and alkyl phosphate type, especially, of course, those customarily used in shampoo compositions, for example, the known C₁₀-C₁₈-alkyl sulfates, and in particular the respective ether sulfates, for example, C₁₂-C₁₄-alkyl ether sulfate, lauryl ether sulfate, especially with 1 to 4 ethylene oxide groups in the molecule, monoglyceride (ether) sulfates, fatty acid amide sulfates obtained by ethoxylation and subsequent sulfatation of fatty acid alkanolamides, and the alkali salts thereof, as well as the salts of long-chain mono- and dialkyl phosphates constituting mild, skin-compatible detergents.

Additional anionic surfactants useful within the scope of the invention are a-olefin sulfonates or the salts thereof, and in particular alkali salts of sulfosuccinic acid semiesters, for example, the disodium salt of monooctyl sulfosuccinate and alkali salts of long-chain monoalkyl ethoxysulfosuccinates.

Suitable surfactants of the carboxylate type are alkyl polyether carboxylic acids and the salts thereof of the formula

R₁-(C₂H₄O)ₙ-O-CH₂COOX

wherein R₁ is a C₈-C₂₀-alkyl group, preferably a C₁₂-C₁₄-alkyl group, n is a number from 1 to 20, preferably 2 to 17, and X is H or preferably a cation of the group sodium, potassium, magnesium and ammonium, which can optionally be hydroxyalkyl-substituted, as well as alkyl amido polyether carboxylic acids of the general formula 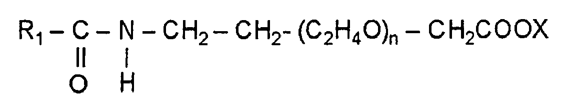 wherein R₁ and X have the above meanings, and n is in particular a number from 1 to 10, preferably 2.5 to 5.

Such products have been known for some time and are on the market, for example, under the trade name AKYPO and AKYPO-SOFT.

Also useful are C₈-C₂₀-acyl isethionates, alone or in admixture with other anionic surfactants, as well as sulfofatty acids and the esters thereof.

It is also possible to use mixtures of several anionic surfactants, for example, a mixture of a C₁₂ - C₁₄ alkyl ether sulphate and a sulfosuccinate, preferably in a proportion of 1 : 3 to 3 : 1, or of an ether sulfate and a polyether carboxylic acid or alkyl amidoether carboxylic acid.

An overview of the anionic surfactants used in liquid body cleansing compositions can furthermore be found in the monography of K. Schrader, "Grundlagen und Rezepturen der Kosmetika", 2nd Ed.(1989, Hüthig Buchverlag), pp. 595-600 and pp. 683 to 691.

Further suitable anionic surfactants are also C₈-C₂₂-acyl aminocarboxylic acids or the water-soluble salts thereof. Especially preferred is N-lauroyl glutamate, in particular as sodium salt, as well as, for example, N-lauroyl sarcosinate, N-C₁₂-C₁₈-acyl asparaginic acid, N-myristoyl sarcosinate, N-oleoyl sarcosinate, N-lauroyl methylalanine, N-lauroyl lysine and N-lauroyl aminopropyl glycine, preferably in form of the water-soluble alkali or ammonium, in particular the sodium salts thereof, preferably in admixture with the above-named anionic surfactants.

Further surfactants in the shampoo compositions according to the invention are nonionic surfactants in admixture with anionic surfactants.

These are described in Schrader, I.c., on pages 600-601 and pp. 694-695. Especially suited are alkyl polyglucosides of the general formula

R₂-O-(R₃O)ₙO-Zₓ

wherein R₂ is an alkyl group with 8 to 18 carbon atoms, R₃ is an ethylene or propylene group, Z is a saccharide group with 5 to 6 carbon atoms, n is a number from 0 to 10 and x is a number between 1 and 5.

These alkyl polyglucosides have recently become known in particular as excellent skin-compatible, foam improving agents in liquid detergents and body cleansing compositions, and are present in an amount from about 1 % to 15 %, in particular from 1 % to 10 % by weight, calculated to the total composition.

Mixtures of anionic surfactants and alkyl polyglucosides as well as the use thereof in liquid body cleansing compositions are already known, for example, from EP-A 70 074. The alkyl polyglucosides disclosed therein are basically also suited within the scope of the present invention; as well as the mixtures of sulfosuccinates and alkyl polyglucosides disclosed in EP-A 358 216.

Further nonionic surfactant components are, for example, long-chain fatty acid monoand dialkanolamides, such as coco fatty acid monoethanolamide and myristic fatty acid monoethanolamide, which can also be used as foam enhancers, preferably in amounts from 1 % to 5 % by weight.

Further additionally useful nonionic surfactants are, for example, the various sorbitan esters, such as polyethylene glycol sorbitan stearic acid ester, fatty acid polyglycol esters or poly-condensates of ethyleneoxide and propyleneoxide, as they are on the market, for example, under the trade name "Pluronics^{R}", as well as fatty alcohol ethoxylates.

Further suitable nonionic surfactants are amineoxides in an amount from 0.25 % to 5 %, preferably 0.5 % to 3.5 % by weight, calculated to the total composition.

Such amineoxides are state of the art, for example C₁₂-C₁₈- alkyl dimethyl amineoxides such as lauryl dimethyl amineoxide, C₁₂-C₁₈- alkyl amidopropyl or -ethyl amineoxides, C₁₂-C₁₈ -alkyl di(hydroxyethyl) or (hydroxypropyl) amineoxides, or also amineoxides with ethyleneoxide and/or propyleneoxide groups in the alkyl chain. Such amineoxides are on the market, for example, under the trade names "Ammonyx^{â}", "Aromox^{â}" or "Genaminox^{â}".

Further nonionic surfactants useful in the compositions according to invention are C₁₀-C₂₂-fatty alcohol ethoxylates. Especially suited are C₁₀-C₂₂-fatty alcohol ethers, the alkyl polyglycol ethers known by the generic terms "Laureth", "Myristeth", "Oleth", "Ceteth", "Deceth", "Steareth" and "Ceteareth" according to the CTFA nomenclature, including addition of the number of ethylene oxide molecules, e.g., "Laureth-16": The average degree of ethoxylation thereby ranges between 2.5 and 25, preferably 10 and 20.

As further surfactant component, the compositions according to the invention can also contain amphoteric or zwitterionic surfactants, for example in an amount from 0.5 % to 10 %, preferably from 1 % to 7.5 % by weight, calculated to the total composition. It has especially been found out that addition of zwitterionic or amphoteric surfactants enhances foam feeling in terms of creaminess, foam volume and as well as skin compatibility is improved. For achieving milder formulations anionic surfactant, especially of sulphate types, to amphoteric surfactant ratio should be in the range of 10:1 to 1:1, preferably 5:1 to 1:1.

Useful as such are in particular the various known betaines such as alkyl betaines, fatty acid amidoalkyl betaines and sulfobetaines, for example, lauryl hydroxysulfobetaine; long-chain alkyl amino acids, such as cocoaminoacetate, cocoaminopropionate and sodium cocoamphopropionate and -acetate have also proven suitable.

In detail, it is possible to use betaines of the structure 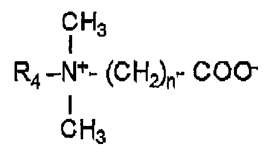 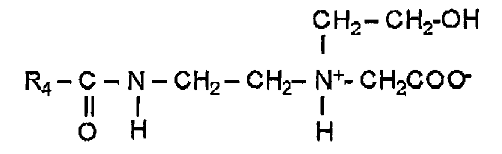 wherein R₄ is a C₈-C₁₈-alkyl group and n is 1 to 3; sulfobetaines of the structure 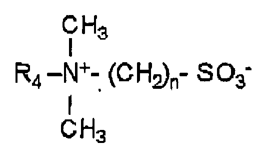 wherein R₄ and n are same as above;
and amidoalkyl betaines of the structure 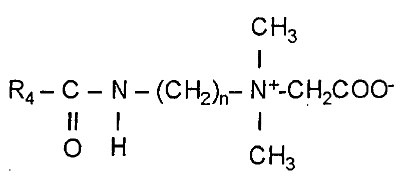 wherein R₄ and n are same as above.

Hair conditioning compositions of the present invention can be in the form of either leave in or rinse of emulsions used especially after shampooing. The emulsion type conditioners comprise in addition to blue lotus flower extract at least one fatty alcohol of the following formula

R₅-OH

where R₅ is a saturated or unsaturated, branched or non-branched fatty acyl chain with 8 - 24 C atoms. Concentration of fatty alcohols is usually less than 20%, preferably less than 15% by weight calculated to total composition. Typical examples to the most useful fatty alcohols are myristyl alcohol, palmityl alcohol, cetyl alcohol, stearyl alcohol, behenyl alcohol and their mixtures. As a mixed fatty alcohol the mostly used one is the cetearyl alcohol as well preferred in the compisions of the present invention.

The hair conditioning composition of the present invention comprises surface-active substances as emulsifiers. These can be anionic and/or nonionic and/or cationic and/or amphoteric or zwitterionic and/or their mixtures incorporated at a concentration ranging between 0.1 - 10 %, preferably 0.1 - 7.5% and more preferably 0.1 - 5% by weight calculated to the total composition. Preferred emulsifiers are of non-ionic and cationic types and the above-specified concentrations are given for these emulsifiers. The anionic ones are as a rule not preferred and if their presence is desirable because of any reason, those should form the very minor part, as electrostatic interactions with the cationic material can disturb the stability of those conditioners. Zwitterionic ones are the ones preferred to lesser extent. Structural information and examples to the anionic, non-ionic and amphoteric ones are given above for shampoo preparations is as well valid for the emulsion type of conditioners.

The compositions of the present invention comprise cationic hair conditioning agents. Cationic conditioning agents is selected from cationic amphiphilic compounds, cationic polymers and cationic silicone derivatives. The compositions can as well comprise other conditioning agents such as oily substances and non-ionic substances.

Cationic surfactants are useful in the compositions of the present invention as emulsifiers and as conditioning agents at the same time represented with the general formula below: 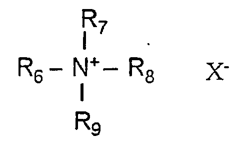 where R₆ is a saturated or unsaturated, branched or non-branched alkyl chain with 8-22 C atoms or

R₁₀ CO NH (CH₂)ₙ

where Rio is saturated or unsaturated, branched or non-branched alkyl chain with 7-21 C atoms and n has typical value of 0 - 4 or

R₁₁ CO O (CH₂)ₙ

where R₁₁ is saturated or unsaturated, branched or non-branched alkyl chain with 7-21 C atoms and n has typical value of 0 - 4, and
R₇ is a hydrogen, lower alkyl chain with 1 to 4 carbon atoms, saturated or unsaturated, branched or non-branched alkyl chain with 8-22 C atoms or

R₁₀ CO NH (CH₂)ₙ

where R₁₀ is saturated or unsaturated, branched or non-branched alkyl chain with 7-21 C atoms and n has typical value of 0 - 4 or

R₁₁ CO O (CH₂)ₙ

where R₁₁ is saturated or unsaturated, branched or non-branched alkyl chain with 7-21 C atoms and n has typical value of 0 - 4, and R₈ and R₉ are independent from each other H or lower alkyl chain with 1 to 4 carbon atoms, and X is chloride, bromide or methosulfate.

It should be noted that quaternary ammonium compounds with single alkyl chain are preferred as emulsifiers as well. This should certainly not mean that those are excluded to be used as conditioning ingredients. With the above it is especially stated that the single alkyl chain cationic surfactants have emulsifying ability as well. The others, such as di alkyl dimonium chloride, is more preferred as conditioner. Suitable cationic surfactants and or conditioning agents are, for example, long-chain quaternary ammonium compounds which can be used alone or in admixture with one another, such as cetyl trimethyl ammonium chloride, myristoyl trimethyl ammonium chloride, trimethyl cetyl ammonium bromide, stearyl trimethyl ammonium chloride, dimethyl stearyl ammonium chloride, dimethyl dihydrogenated tallow ammonium chloride, stear trimonium chloride, dipalmitoyl dimonium chloride, distearyl dimethyl ammonium chloride, stearamidopropyl trimonuim chloride, dioleoylethyl dimethyl ammonium methosulfate.

From the above quaternary ammonium compounds disclosed with the general formula, especially preferred are those compounds known per se and are on the market, for example, under the trade names "Schercoquat^{®}", "Dehyquart^{®} F30" and "Tetranyl^{®}". Use of these compounds, the so-called "esterquats", in hair care compositions is described, for example, in WO-A 93/107 48, WO-A 92/068 99 and WO-A 94/166 77, wherein, however, there is no reference made to the combinations according to the present invention and the advantageous properties thereof.

Again from the above quaternary ammonium compounds disclosed with the general formula, especially preferred are these compounds are known per se and on the market, for example, under the trade name "INCROQUAT^{â} HO" or "OCS". Those compounds known with a general ingredient category under "amidoquat" in the cosmetic industry.

Composition of the present invention comprises cationic polymers as conditioning agents. Those are cationic cellulose type polymers know as Polymer JR type from Amerchol such as Polyquaternium 10 or cationic guar gum known with trade name Jaguar from Rhône-Poulenc and chemically for example Guar hydroxypropyl trimonium chloride. Furthermore, chitosan and chitin can also be included in the compositions as cationic natural polymers.

Furthermore, it has especially been found suitable those cationic polymers known with their CTFA category name Polyquaternium. Typical examples of those Polyquaternium 6, Polyquaternium 7, Polyquaternium 10, Polyquaternium 11, Polyquaternium 16, Polyquaternium 22 and Polyquaternium 28, Polyquaternium 30, Polyquaternium 37, Polyquaternium 36, Polyquaternium 46.

As well those polymers known with their CTFA category name Quaternium are suitable. Those are for example Quaternium-8, Quaternium-14, Quaternium-15, Quaternium-18, Quaternium-22, Quaternium-24, Quaternium-26, Quaternium-27, Quaternium-30, Quaternium-33, Quaternium-53, Quaternium-60, Quaternium-61, Quaternium-72, Quaternium-78, Quaternium-80, Quaternium-81, Quaternium-81, Quaternium-82, Quaternium-83 and Quaternium-84.

In this context, reference is also made to the cationic polymers disclosed in DE 25 21 960, 28 11 010, 30 44 738 and 32 17 059, as well as to the products described in EP-A 337 354 on pages 3 to 7. It is also possible to use mixtures of various cationic polymers.

The cationic polymers also include the quaternized products of graft polymers from organopolysiloxanes and polyethyl oxazolines described in EP-A 524 612 and EP-A 640 643.

Cationic conditioning agents are used alone or in combination with each other. As a results a composition falling in the scope of the present invention can contain for example single cationic polymer as the sole conditioner as well can contain cationic polymer, cationic surfactant and cationic silicone derivative in the same composition as conditioning compounds.

Typical concentration range for cationic conditioners mentioned above can be 0.01 - 7.5% by weight, preferably 0.05 - 5% by weight calculated to the total composition. Oily substances as conditioners according to the present invention are selected from silicone oils either volatile or non-volatile, natural and synthetic oils. Among silicone oils those can be added to the compositions include dimethicone, dimethiconol, polydimethylsiloxane, DC fluid ranges from Dow Corning.

Natural oils suitable are such as olive oil, almond oil, avocado oil, weizenkeim oil, ricinus oil, coconut oil, palm oil, sesame oil, peanut oil, whale oil, sunflower oil, peach kernel oil, wheat germ oil, macadamia nut oil, night primrose oil, jojoba oil, castor oil, or soya oil, lanolin and the derivatives thereof, as well as mineral oils such as paraffin oil and petrolatum.

Lipophilic compounds such as fatty acid esters are as well suitable for the composition of the present invention. Those are such as isopropyl myristate, palmitate, stearate and isostearate, oleyl oleate, isocetyl stearate, hexyl laurate, dibutyl adipate, dioctyl adipate, myristyl myristate, oleyl erucate, polyethylene glycol and polyglyceryl fatty acid esters such as PEG-7-glyceryl cocoate, cetyl palmitate, etc.. Non-ionic conditioning agents may be polyols such as glycerin, glycol and derivatives, polyethyleneglycoles known with trade names Carbowax PEG from Union Carbide and Polyox WSR range from Amerchol, polyglycerin, polyethyleneglycol mono or di fatty acid esters having general formula

R₁₂ CO (OCH₂CH₂)ₙ OH

R₁₂ CO (OCH₂CH₂)ₙ O OC R₁₃

where R₁₂ and R₁₃ are independent from each other saturated, unsaturated or branched or non-branched alkyl chain with 7 to 21 C atoms and n is typically 2-100.

Typical concentration range for any of the additional conditioners mentioned above other than cationic conditioning compounds can be 0.01 - 15% by weight, preferably 0.05 - 5% by weight calculated to the total composition.

The compositions according to the invention can also comprise further agents, such as protein hydrolyzates and polypeptides, e.g. keratin hydrolyzates, collagen hydrolyzates of the type "Nutrilan" or elastin hydrolyzates, as well as, in particular vegetable, optionally cationized protein hydrolyzates, for example "Gluadin". Additional natural plant extracts can as well form part of the compositions of the present invention. Those are incorporated usually in an amount of about 0.01 % to about 10 %, preferably 0.05 % to 7.5 %, in particular 0.1 % to 5 % by weight, calculated as dry residue thereof to the total composition. Suitable aqueous (e.g. steam-distilled) alcoholic or hydro-alcoholic plant extracts known per se are in particular extracts from leaves, fruits, blossoms, roots, rinds or stems of aloe, pineapple, artichoke, arnica, avocado, valerian, bamboo, henbane, birch, stinging nettle, echinacea, ivy, wild angelica, gentian, ferns, pine needles, silver weed, ginseng, broom, oat, rose hip, hamamelis, hay flowers, elderberry, hop, coltsfoot, currants, chamomile, carrots, chestnuts, clover, burr root, cocoanut, cornflower, lime blossom, lily of the valley, marine algae, balm, mistletoe, passion flower, ratanhia, marigold, rosemary, horse chestnut, pink hawthorn, sage, horsetail, yarrow, primrose, nettle, thyme, walnut, wine leaves, white hawthorn, etc.

Suitable trade products are, for example, the various "Extrapon" products, "Herbasol^{R} ", "Sedaplant^{R}" and "Hexaplant^{R}". Extracts and the preparation thereof are also described in "Hagers Handbuch der pharmazeutischen Praxis", 4^{th} Ed.

Among the natural ingredients in the form of an extract, especially preferred in addition to the blue lotus flower extract component of the composition according to the invention is green tea extract. This tea extract is obtained from the leaves, leaf buds and tender stems of the tea shrub, Camellia sinensis or Camellia oleifera, by aqueous or hydro-alcoholic extraction and subsequent spray-drying. In difference to black tea, green tea is a non-fermented product obtained from the Thea sinensis or Thea assamica species. An overview of the biological and pharmacological effects of green tea and the ingredients thereof can be found, e.g., in an article by A. Pistorius, "Seifen-Öle-Fette-Wachse-Journal", Volume 122., No. 7/1996, pages 468 to 471, to which reference is made. The content of green tea extract is variable in the compositions according to the invention. It preferably ranges from 0.01 % to 10 %, preferably 0.05 % to 5% by weight, calculated to the total composition and the pulverulent extract.

The compositions may contain organic solvents such as ethanol. propanol, isopropanol, benzyl alcohol, benzyloxyethanol, alkylene carbonates such as ethylene carbonate and propylene carbonate, phenoxyethanol, butanol, isobutanol, cyclohexane, cyclohexanol, hexyleneglycol, ethylenecarbonate, ethyleneglycol monoethylether, ethylene glycol monobutyl ether, ethylene glycol monophenyl ether, 1-phenylethylalcohol, 2-phenylethylalcohol, o-methoxyphenol. Concentration of organic solvents in the composition should not exceed 5% by weight. It should be noted that penetration enhancers are useful for both cleansing and after shampoo conditioning preparations. It is obvious that the concentration in the cleansing compositions is usually lower than in the conditioning preparations.

Compositions of the present invention can comprise UV filters either for stabilization of the product colour or for protection of hair from environmental influences such as loss of elasticity, loss of hair colour (bleaching effect of sun light). The UV-absorbing substance is preferably selected from the following compounds: 4-Aminobenzoic acid and the esters and salts thereof, 2-phenyl benzimidazole-5-sulfonic acid and the alkali and amine salts thereof, 4-dimethyl aminobenzoic acid and the esters and salts thereof, cinnamic acid and the esters and salts thereof, 4-methoxycinnamic acid and the esters and salts thereof, salicylic acid and the esters and salts thereof, 2.4-dihydroxybenzophenone, 2.2'.4.4'-tetrahydroxy- benzophenone, 2-hydroxy-4-methoxybenzophenone and its 5-sulfonic acid or the sodium salt thereof, 2.2'-dihydroxy-4.4'-dimethoxybenzophenone, 2-hydroxy-5-chlorobenzophenone, 2.2'-dihydroxy-4-methoxybenzophenone, 2.2'-dihydroxy-4.4'-dimethoxy-5.5'-disulfobenzo-phenone or the sodium salt thereof, 2-hydroxy-4-octyloxybenzophenone, 2-hydroxy-4-methoxy-4'-methylbenzophenone, 3-benzyl-idenecampher, 3-(4'-sulfo)-benzyl-idenebornane-2-one and the salts thereof and/or 3-(4'-methyl benzylidene)-DL-campher.

The preferred amount of the UV-absorber ranges from about 0.01 % to 2.5%, more preferably from 0.05 % to 1 % by weight, calculated to the total composition.

Composition of the present invention especially those of cleansing types can be transparent as well a pearly. Transparency of the composition is judged by naked eye in a transparent shampoo bottle with a thickness not more than 5 cm. In the case a transparent appearance is wished, the following ingredients are not essential. However, pearl-shiny appearance is achieved with those dispersed in shampoo compositions in crystalline form, i.e. so called pearl-shine agents. The preferred once are PEG-3 distearate and ethylene glycol distearate. The concentration of those can typically be from 0.1 to 3%, preferably 0.5 to 2% by weight, calculated to the total composition.

Solubilizers may be added to the compositions especially when oily substances are chosen as conditioning agents and fragrance oils with highly lipophilic properties. Typical solubilizers may be hydrogenated castor oil known with the trade mark Cremophor RH series from BASF. It should be noted that as well the surfactant mixture can be a good solubilizer for fragrance oils. Typical concentration of the solubilizers can be in the range of 0.01 - 2% by weight, preferably 0.1-1% by weight, calculated to the total composition. It should be noted without limiting their use that the solubilizers are especially used in the clear cleansing and/or conditioning preparations in order to overcome the turbidity caused by addition of lipophilic materials.

Antidandruff agents such as piroctone olamine (Octopirox), zinc pyrrithion, climbazol can as well be used when an antidandruff effects is targeted. Typical useful concentration range for antidandruff agents is between 0.1 - 2% by weight calculated to the total composition.

The compositions of the present invention can comprise hair-restructuring agents. The hair restructuring agents preferred are especially the ones disclosed in the German patent DE 197 51 550 C2.

One of the known hair restructuring agents is ceramide type of compound with the general formula 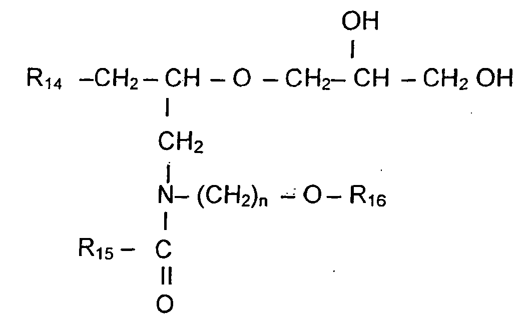 where R¹⁴ and R¹⁵ are independent from each other alkyl- or. alkenyl group with 10 to 22 carbon atoms, R¹⁶ is methyl, ethyl, n-propyl or isopropyl group and n is a number between 1 to 6, preferably 2 or 3.

Another preferred hair restructuring agents are fatty acids with 10 to 24 carbon atoms and especially with 16 to 24 carbon atoms.

Sterols,especially the phytosterols, are as well preferred hair restructuring agents as disclosed in the above mentioned german patent. Especially preferred ones are of plant origin for example ergosterol, sitosterol, stigmasterol, fucosterol, brassicasterol, fungisterol, campesterol, zymosterol, ascosterol, cerevisterol, episterol, faecosterol, spinasterol. Among those phytosterols, the ones found in "Avocadin" which is the unsaponified fraction of the avocado oil is more preferred.

The concentration of ceramide in the compositions of the present invention can be in the range of 0.01 to 2 and especially 0.01 to 1 % by weight calculated to the total weight of the composition. The fatty acids may be contained at a level of 0.01 to 2.5% and epecially 0.01 to 1% by weight calculated to the total weight of the composition. Phytosterol concentration of the conditioners is less than 1% and preferably less than 0.5% by weight calculated to the total weight of the composition. It should be noted without limiting the use of those ingredients the effect of those hair restructuring ingredients is especially elevated when used in combination with penetration enhancers.

The compositions according to the invention can also contain dyestuffs for direct dyeing of human hair, as so-called tinting or dyeing shampoos and conditioners.
The compositions of the present invention shampoo and after shampoo conditioning compositions can as well be colour-enhancing compositions. In this case, direct dyes are included into the compositions. As direct dyes, cationic and neutral nitro dyes are useful. The use of anionic dyes is as well possible though the color enhancing ability especially at higher end of pH specification is observed to be very low. For shampoo compositions at a low pH range, the acidic dyes can as well be preferred. It is also desirable for the best performance not to use the mixture of acidic and cationic dyestuffs in the same shampoo compositions. The concentration of those dyes is in between 0.0001 - 5% by weight, preferably 0.0001 -2.5% by weight and more preferably 0.0001 - 1.5% by weight calculated to the total composition.

The useful cationic dyes, especially preferred in the colouring shampoo and conditioners according to the invention are: Basic Blue 6, Basic Blue 7, Basic Blue 9, Basic Blue 26, Basic Blue 41, Basic Blue 99, Basic Brown 4, Basic Brown 16, Basic Brown 17, Natural Brown 7, Basic Green 1, Basic Orange 31, Basic Red 2, Basic Red 22, Basic Red 51, Basic Red 76, Basic Violet 1, Basic Violet 3, Basic Violet 10, Basic Violet 14, Basic Yellow 57, Basic Yellow 87.

Suitable neutral nitro dyes are HC Blue No.2, HC Blue No.4, HC Blue No.5, HC Blue No.6, HC Blue No.7, HC Blue No.8, HC Blue No.9, HC Blue No.10, HC Blue No.11, HC Blue No.12, HC Blue No.13, HC Brown No.1, HC Brown No.2, HC Green No.1, HC Orange No.1, HC Orange No.2, HC Orange No.3, HC Orange No.5, HC Red BN, HC Red No.1, HC Red No.3, HC Red No.7, HC Red No.8, HC Red No.9, HC Red No.10, HC Red No.11, HC Red No.13, HC Red No.14, HC Violet BS, HC Violet No.1, HC Violet No.2, HC Yellow No.2, HC Yellow No.4, HC Yellow No.5, HC Yellow No.6, HC Yellow No.7, HC Yellow No.8, HC Yellow No.9, HC Yellow No.10, HC Yellow No.11, HC Yellow No.12, HC Yellow No.13, HC Yellow No.14, HC Yellow No.15.

Suitable anionic dyestuffs are, for example: Acid Black 1, Acid Blue 1, Food Blue 5, Acid Blue 9, Acid Blue 74, Acid Red 18, Acid Red 27, Acid Red 87, Acid Red 92, Acid Orange 7, Acid Violet 43, Acid Yellow 1, Acid Yellow 23, Acid Yellow 3, Food Yellow No. 8, D&C Brown No. 1, D&C Green No. 5, D&C Orange No. 4, D&C Orange No. 10, D&C Orange No. 11, D&C Red No. 21, D&C Red No. 27, D&C Red No. 33, D&C Yellow No. 7, D&C Yellow No. 8, FD&C Red No. 4, FD&C Yellow No. 6.

The pH of the compositions according to the invention is in the range of 1.5 to 8, preferably 2 to 7, more preferably 2 to 6. For adjusting the pH of the said compositions, following ingredients can be used: Organic acids such as citric acid, lactic acid, tartaric acid, malic acid, maleic acid, fumaric acid, levulinic acid, butyric acid and hydroxy butyric acids, valeric acid, oxalic acid, succinic acid, mandelic acid, glycolic acid, glucuronic acid, propionic acid, salicylic acid or acetic acid or inorganic acids such as hydrochloric acid, phosphoric acid, sulphuric acid, nitric acid. Concentration of the organic and/or inorganic acids or their mixtures should be chosen in a way that composition reaches the desired pH value as given above. Typically concentration for acids can be 0.01 - 3% by weight, preferably 0.05 - 2% by weight, more preferably 0.05 - 1.5% by weight calculated to the total composition. The pH of the composition can also be adjusted to the required pH by using alkaline solution such as sodium hydroxide, potassium hydroxide or their salts with those acids mentioned above in the case that at the selected acid concentration pH of the composition is lower than that of the aimed value.

The viscosity of the compositions depends on the type of application according to the invention. Compositions for cleansing purposes, conventional shampoos have the viscosity in the range of between about 1000 and about 10,000 mPa.s at 20°C, measured according to Brookfield or Höppler at a shear rate of 10 sec⁻¹. Whereas cleansing compositions dispensed form an aerosol and/or pump foamer should preferably be very liquid, i.e. viscosity values more than approximately 200 mPa.s measured as given above are not appropriate.

Viscosity of the conditioner or after shampoo compositions can vary largely. Those can be very thick pasty emulsions and as well very liquid compositions to be sprayed onto hair wit the aid of a mechanical spray. An aerosol spray composition is as well suitable application form. The after shampoo composition can also be gel type of preparation. Therefore, viscosity of compositions should be chosen appropriately which allows the application onto hair.

In general the compositions of the present invention can be in the form of fully transparent, semitransparent or non-transparent. Transparency should certainly be judged by naked eye in a therefore suitable vessel.

Furthermore compositions of the present invention can comprise all substances customarily found in such preparations. Examples of such substances are complexing agents, dyestuffs, preservatives, pH-regulants, viscosity regulants such as inorganic salts or polymers to the extent they are not already contained in the initial surfactant mixtures, fragrances, pearl-gloss agents, thickening agents, moisturizers, etc. Furthermore, compositions dispensed from an aerosol preferably do not contain any preservative.

The following examples are to illustrate the invention, but not to limit it.

In all example below, the blue lotus extract used was consisting of 80% propyleneglycol, 10% blue lotus flower and 10% water. The pH of the extract was 6.2, refractive index at 20°C was 1.428 and density at 20°C was 1.050 g/cm³. In the example the amount of extract is referred to and not the dry matter. The dry matter can simply be calculated by taking the 10% of the extract.

### Example 1

### Shampoo composition

| | % by weight |
|---|---|
| Sodium lauryl ether sulfate (2.5EO) | 10.0 |
| C₁₂-C₁₄-alkyl polyglucoside (P.D.: 1.5) | 3.0 |
| Cocamidopropyl betaine | 5.0 |
| Blue lotus flower extract | 2.0 |
| Polyquaternium-10 | 0.4 |
| PEG-55-1,2-propyleneglycol oleate | 1.0 |
| Panthenol | 0.5 |
| Sodium chloride | q.s. to 5000 mPa.s* |
| Preservative, fragrance | q.s. |
| Citric acid | q.s. to pH 5.0 |
| Water | to 100 |
| * measured as given in the description. | |

Shampoo composition is prepared by combining first the surfactants and other ingredients were added afterwards to the surfactant mixture except that polymer is first dissolved in water before adding to the surfactant mixture.

The shampoo according to the invention developed a full, creamy lather and provided the hair with good wet and dry combability, volume, full and soft body.

The shampoo was tested in a half side test against a shampoo composition not comprising blue lotus flower extract with 10 female volunteers According to evaluation of hair dresser the overall preference was with 70% on the side washed with shampoo composition with the above composition comprising 2% blue lotus flower extract. In shine evaluation in 80% of the cases the side with blue lotus flower extract comprising shampoo composition washed was preferred. In the remaining 20% no difference was found. This result clearly show that blue lotus flower extract improves hair shine and overall conditioning of hair.

### Example 2

### Fine hair shampoo

| | % by weight |
|---|---|
| Sodium lauryl ether sulfate (2.5EO) | 6.5 |
| C₁₂-C_{I4}-alkyl polyglucoside (P.D.: 1.5) | 3.0 |
| Cocamidopropyl betaine | 2.0 |
| Sodium lauroyl glutamate | 1.5 |
| Blue lotus flower extract | 2.0 |
| Polyquaternium-6 | 0.3 |
| PEG-55-1,2-propyleneglycol oleate | 2.0 |
| Sodium chloride | q.s. to 4000 mPa.s* |
| Preservative, fragrance | q.s. |
| Citric acid | q.s to pH 5.5 |
| Water | to 100 |
| * measured as given in the description. | |

In a half side test against a composition not comprising an blue lotus flower extract, it was found out that the above composition showed superior effects in volume and body and hair shine was found to be excellent.

### Example 3

### Antidandruff shampoo

| | % by weight |
|---|---|
| Sodium lauryl ether sulfate (2.5EO) | 10.0 |
| C₁₂-C₁₄-alkyl polyglucoside (P.D.: 1.5) | 3.0 |
| Cocamidopropyl betaine | 5.0 |
| Sodium lauroyl glutamate | 2.0 |
| Blue lotus flower extract | 2.0 |
| Piroctane olamine | 1.0 |
| Polyquaternium-6 | 0.5 |
| PEG-55-1,2-propyleneglycol oleate | 0.5 |
| Sodium chloride | q.s. to 4500 mPa.s* |
| Preservative, fragrance | q.s. |
| Citric acid | q.s. to pH 5.0 |
| Water | to 100 |
| * measured as given in the description. | |

In addition to an outstanding anti-dandruff effect and excellent foaming properties, this shampoo showed good hair-conditioning properties and excellent hair shine improvement performance.

### Example 5

### Conditioning composition - rinse-off

| | % by weight |
|---|---|
| Cetylstearylalcohol | 5.0 |
| Di-C₁₂-C₁₅-alkyldimethylammoniumchlorid | 1.0 |
| Stearyltrimethylammoniumchlorid | 2.0 |
| 1,2-Propandiol | 2.0 |
| Blue lotus flower extract | 2.0 |
| Preservative, fragrance | q.s. |
| Citric acid | q.s. to pH 4.0 |
| Water | to 100 |

Hair treated with the above formulation is judged to have more elasticity, volume and body and especially excellently high shine. Exclusion of Blue lotus flower extract resulted in less smoothness and therefore as well less shine.

### Example 6

### Spray conditioner - non-aerosol

| | % by weight |
|---|---|
| Ethanol | 45.0 |
| Polyquaternium-6 | 0.1 |
| Ceramide compund according to formula (R¹⁴=C₁₅H₃₁; R¹⁵=C₁₆H₃₃; R¹⁶=H; n=2) | 0.1 |
| Behenic acid | 0.2 |
| Avocadin | 0.05 |
| PEG-160-hydrogenated castor oil | 0.5 |
| Blue lotus flower extract | 1.0 |
| Fragrance | 0.15 |
| Citric acid | q.s. to pH 4.7 |
| Water | to 100 |

The composition is applied to hair from a common pump spray. Hair treated with the composition has more elasticity, smoothness and shine. Leaving out Blue lotus flower extract resulted in loss of smoothness and shine. Upon repeated application hair structure improvements are as well observed. This is due to the content of restructuring ingredients.

### Example 7

### Foam conditioner

| | % by weight |
|---|---|
| Quaternium-80 | 0.2 |
| Polyquaternium-11 | 0.7 |
| Propylene gylcol | 5.0 |
| Ceramide compund according to formula (R¹⁴=C₁₅H₃₁; R¹⁵=C₁₆H₃₃; R¹⁶=H; n=2) | 0.2 |
| Behenic acid | 0.2 |
| Avocadin | 0.1 |
| PEG-160-hydrogenated castor oil | 0.5 |
| Blue lotus flower extract | 1.5 |
| Fragrance | 0.15 |
| Citric acid | q.s. to pH 5.0 |
| Water | to 100 |

The composition is filled in an aerosol can at a ratio of 90:10 with commonly used propellant, propan/butan mixture. The composition showed excellent hair conditioning properties. It should be noted that the composition can be used in two different ways, leave-in and rinse off. In leave-in application, amount used is obviously less than in the case of a rinse of application

### Example 8

### Colour enhancing shampoo

| | % by weight |
|---|---|
| Sodium lauryl ether sulfate (2.5EO) | 5.0 |
| C₁₂-C₁₄-alkyl polyglucoside (P.D.: 1.5) | 5.0 |
| Cocamidopropyl betaine | 5.0 |
| Blue lotus flower extract | 2.0 |
| Polyquaternium-10 | 0.4 |
| PEG-55-1,2-propyleneglycol oleate | 1.0 |
| Panthenol | 0.5 |
| Basic red 51 | 0.1 |
| Sodium chloride | q.s. to 5000 mPa.s* |
| Preservative, fragrance | q.s. |
| Citric acid | q.s. to pH 5.0 |
| Water | to 100 |
| * measured as given in the description. | |

Application of this shampoo with its good lather and conditioning properties resulted in excellent shiny red reflexes on a dark blonde hair.

### Example 9

### Colouring conditioner - rinse-off

| | % by weight |
|---|---|
| Cetylstearylalcohol | 5.0 |
| Di-C₁₂-C₁₅-alkyldimethylammoniumchlorid | 1.0 |
| Stearyltrimethylammoniumchlorid | 2.0 |
| 1,2-Propandiol | 2.0 |
| Blue lotus flower extract | 2.0 |
| Basic red 51 | 0.1 |
| Preservative, fragrance | q.s. |
| Citric acid | q.s. to pH 4.0 |
| Water | to 100 |

Hair treated with the above formulation is judged to have more smoothness in wet and dry stage, more elasticity, volume and body and especially excellently high shine. Excellent red reflexes were noted on a natural light brown hair. Exclusion of Blue lotus flower extract resulted in less smoothness and therefore as well as less shine.

## Claims

1. Conditioning composition for hair **characterised in that** it comprises Blue lotus (Nymphaea caerulea) flower extract.

2. Composition according to claim 1 **characterised in that** the blue lotus flower extract comprises as solvent a mixture of propylene glycol and water,

3. Composition according to claims 1 and 2 **characterised in that** it comprises blue lotus flower extract at a concentration of 0.01 to 3% by weight, calculated to the total composition and as dry matter of the extract.

4. Composition according to claims 1 to 3 **characterised in that** it comprises additionally at least one cationic hair conditioning agent.

5. Composition according to claim 4 **characterised in that** the cationic conditioning agent is selected from cationic polymers, cationic amphiphilic compounds and cationic silicone and their derivatives.

6. Composition according to claims 4 and 5 **characterised in that** the cationic conditioning agent is cationic amphiphilic compound according to general formula 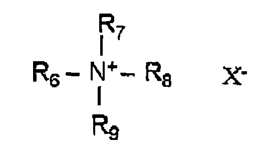 where R₆ is a saturated or unsaturated, branched or non-branched alkyl chain with 8-22 C atoms or
R₁₀ CO NH (CH₂)ₙ
where R₁₀ is saturated or unsaturated, branched or non-branched alkyl chain with 7-21 C atoms and n has typical value of 0 - 4 or
R₁₁ CO O (CH₂)ₙ
where R₁₁ is saturated or unsaturated, branched or non-branched alkyl chain with 7-21 C atoms and n has typical value of 0 - 4, and
R₇ is a hydrogen, lower alkyl chain with 1 to 4 carbon atoms, saturated or unsaturated, branched or non-branched alkyl chain with 8-22 C atoms or
R₁₀ CO NH (CH₂)ₙ
where R₁₀ is saturated or unsaturated, branched or non-branched alkyl chain with 7-21 C atoms and n has typical value of 0 - 4 or
R₁₁ CO O (CH₂)ₙ
where R₁₁ is saturated or unsaturated, branched or non-branched alkyl chain with 7-21 C atoms and n has typical value of 0 - 4, and R₈ and R₉ are independent from each other H or lower alkyl chain with 1 to 4 carbon atoms, and X is chloride, bromide or methosulfate.

7. Conditioning composition according to any of the preceding claims **characterised in that** it is a cleansing composition and comprises additionally at least one surfactant selected from anionic, non-ionic, amphoteric or zwitterionic ones.

8. Conditioning composition according to claims 1 to 6 **characterised in that** the conditioning composition is an emulsion and comprises at least one fatty alcohol and at least one emulsifier.

9. Conditioning composition according to any of the preceding claims **characterised in that** it is a colour enhancing composition and comprises additionally at least one direct dye.

10. Conditioning composition according to claims 4 to 9 **characterised in that** it comprises cationic conditioning agents at a concentration of 0.01 to 7.5% by weight calculated to the total composition.

11. Conditioning composition according to any of the preceding claims **characterised in that** it comprises at least one UV filter.

12. Conditioning composition according to any of the preceding claims **characterised in that** it comprises at least one antidandruff agent,

13. Use of compositions according to any of the preceding claims for improving hair shine.

## Patentansprüche

1. Konditionierende Zusammensetzung für Haare, **dadurch gekennzeichnet, dass** sie Blue Lotus (Nymphaea caerulea) Blumenextrakt enthält.

2. Zusammensetzung nach Anspruch 1, **dadurch gekennzeichnet, dass** der Blue Lotus - Blumenextrakt als Lösungsmittel eine Mischung aus Propylenglykol und Wasser enthält.

3. Zusammensetzung nach den Ansprüchen 1 und 2, **dadurch gekennzeichnet, dass** sie Blue Lotus - Blumenextrakt in einer Menge von 0,01 bis 3 Gew.- %, berechnet auf die Gesamtzusammensetzung und als Trockensubstanz des Extrakts, enthält.

4. Zusammensetzung nach den Ansprüchen 1 bis 3, **dadurch gekennzeichnet, dass** sie zusätzlich mindestens einen kationischen konditionierenden Wirkstoff enthält.

5. Zusammensetzung nach Anspruch 4, **dadurch gekennzeichnet, dass** der kationische konditionierende Wirkstoff aus kationischen Polymeren, kationischen amphiphilischen Verbindungen und kationischen Silikonen und ihren Derivaten ausgewählt ist.

6. Zusammensetzung nach den Ansprüchen 4 und 5, **dadurch gekennzeichnet, dass** das kationische Koditionierungsmittel eine amphiphilische Verbindung ist, entsprechend der allgemeinen Formel 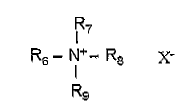 wobei R₆ eine gesättigte oder ungesättigte, verzweigte oder nicht verzweigte Alkylkette mit 8 - 22 Kohlenstoffatomen ist, oder
R₁₀ CO NH (CH₂)ₙ
wobei R₁₀ eine gesättigte oder ungesättigte, verzweigte oder nicht verzweigte Alkylkette mit 7 - 21 Kohlenstoffatomen ist und n einen typischen Wert von 0 - 4 hat, oder
R₁₁ CO O (CH₂)ₙ
wobei R₁₁ eine gesättigte oder ungesättigte, verzweigte oder nicht verzweigte Alkylkette mit 7 - 21 Kohlenstoffatomen ist und n einen typischen Wert von 0 - 4 hat, und
R₇ ein Wasserstoff, eine niedrige Alkylkette mit 1 - 4 Kohlenstoffatomen, eine gesättigte oder ungesättigte, verzweigte oder nicht verzweigte Alkylkette mit 8 - 22 Kohlenstoff ist, oder
R₁₀ CO NH (CH₂)ₙ
wobei R₁₀ eine gesättigte oder ungesättigte, verzweigte oder nicht verzweigte Alkylkette mit 7-21 Kohlenstoffatomen ist und n einen typischen Wert von 0 - 4 hat, oder
R₁₁ CO O (CH₂)ₙ
wobei R₁₁ eine gesättigte oder ungesättigte, verzweigte oder nicht verzweigte Alkylkette mit 7 - 21 Kohlenstoffatomen ist und n einen typischen Wert von 0 - 4 hat, und R₈ und R₉ unabhängig voneinander Wasserstoff oder niedrige Alkylketten mit 1 -4 Kohlenstoffatomen sind, und X Chlorid, Bromid oder Methosulfat ist.

7. Konditionierende Zusammensetzung nach einem der vorhergehenden Ansprüche, **dadurch gekennzeichnet, dass** es sich dabei um ein Reinigungsmittel handelt und zusätzlich mindestens ein Tensid, ausgewählt aus einem anionischen, nichtionischen, amphoterischen oder zwitterionischen Tensid, enthält.

8. Konditionierende Zusammensetzung nach den Ansprüchen 1 bis 6, **dadurch gekennzeichnet, dass** die konditionierende Zusammensetzung eine Emulsion ist und mindestens einen Fettalkohol und mindestens einen Emulgator enthält.

9. Konditionierende Zusammensetzung nach einem der vorhergehenden Ansprüche, **dadurch gekennzeichnet, dass** es sich dabei um ein farbverstärkendes Mittel handelt und zusätzlich mindestens eine direkt ziehende Farbe enthält.

10. Konditionierende Zusammensetzung nach den Ansprüchen 4 bis 9, **dadurch gekennzeichnet, dass** sie kationische konditionierende Wirkstoffe in einer Menge von 0,01 bis 7,5 Gew. -%, berechnet auf die Gesamtzusammensetzung, enthält.

11. Konditionierende Zusammensetzung nach einem der vorhergehenden Ansprüche, **dadurch gekennzeichnet, dass** sie mindestens einen UV-Filter enthält.

12. Konditionierende Zusammensetzung nach einem der vorhergehenden Ansprüche, **dadurch gekennzeichnet, dass** sie mindestens einen Antischuppen-Wirkstoff enthält.

13. Verwendung von Zusammensetzungen nach einem der vorhergehenden Ansprüche zur Verbesserung der Leuchtkraft des Haares.

## Revendications

1. Composition de conditionnement pour cheveux **caractérisé en ce qu'**elle comprend un extrait de fleur de lotus bleu (Nymphaea caerulea).

2. Composition selon la revendication 1, **caractérisée en ce que** l'extrait de fleur de lotus bleu contient comme solvant un mélange de propylèneglycol et d'eau.

3. Composition selon les revendications 1 et 2, **caractérisée en ce qu'**elle comprend un extrait de fleur de lotus bleu à une concentration comprise entre 0,01 et 3 % en poids calculée par rapport à la composition totale et en tant que matière sèche de l'extrait, comprise entre 0,01 et 3 % en poids.

4. Composition selon les revendications 1 à 3, **caractérisée en ce qu'**elle comprend de plus au moins un agent cationique de conditionnement de cheveux.

5. Composition selon la revendication 4, **caractérisée en ce que** l'agent cationique de conditionnement est sélectionné parmi les polymères cationiques, les composés amphiphiles cationiques, les silicones cationique ainsi que leurs dérivés.

6. Composition selon les revendications 4 et 5,
**caractérisé en ce que** l'agent cationique de conditionnement est un composé cationique amphiphile de formule générale : 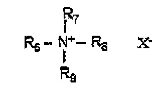 où R₆ est une chaîne d'alkyle saturé ou insaturé, ramifiée ou non ramifiée et comptant de 8 à 22 atomes de C ou
R₁₀CONH(CH₂)ₙ
où R₁₀ est une chaîne d'alkyle saturé ou insaturé, ramifiée ou non ramifiée et comptant de 7 à 21 atomes de C, n étant typiquement compris entre 0 et 4 ou
R₁₁COO(CH₂)ₙ
où R₁₁ est une chaîne d'alkyle saturé ou insaturé, ramifiée ou non ramifiée et comptant de 7 à 21 atomes de C, n étant typiquement compris entre 0 et 4 et
R₇ est un hydrogène, une chaîne d'alkyle inférieur qui compte de 1 à 4 atomes de carbone, une chaîne d'alkyle saturé ou insaturé, ramifiée ou non ramifiée qui compte de 8 à 22 atomes de C ou
R₁₀CONH(CH₂)ₙ
où R₁₀ est une chaîne d'alkyle saturé ou insaturé, ramifiée ou non ramifiée et qui compte de 7 à 21 atomes de C, n étant typiquement compris entre 0 et 4 ou
R₁₁COO(CH₂)ₙ
où R₁₁ est une chaîne d'alkyle saturé ou insaturé, ramifiée ou non ramifiée et qui compte de 7 à 21 atomes de C, n étant typiquement compris entre 0 et 4, R₈ et R₉ représentent indépendamment l'un de l'autre H ou une chaîne d'alkyle inférieur qui compte de 1 à 4 atomes de carbone et X est un chlorure, un bromure ou un méthosulfate.

7. Composition de conditionnement selon l'une quelconque des revendications précédentes, **caractérisée en ce que** c'est une composition de nettoyage et **en ce qu'**elle comprend de plus au moins un agent tensioactif sélectionné dans l'ensemble constitué des agents tensioactifs anioniques, des agents tensioactifs non ioniques, des agents tensioactifs amphotères ou des agents tensioactifs zwitterioniques.

8. Composition de conditionnement selon l'une quelconque des revendications 1 à 6, **caractérisée en ce que** la composition de conditionnement est une émulsion et comprend au moins un alcool gras et au moins un émulsifiant.

9. Composition de conditionnement selon l'une quelconque des revendications précédentes, **caractérisée en ce que** c'est une composition qui renforce la couleur et **en ce qu'**elle comprend de plus au moins un colorant direct.

10. Composition de conditionnement selon les revendications 4 à 9, **caractérisée en ce qu'**elle comprend des agents de conditionnement cationiques à une concentration comprise entre 0,01 et 7,5 % en poids par rapport à la composition totale.

11. Composition de conditionnement selon l'une quelconque des revendications précédentes, **caractérisée en ce qu'**elle comprend au moins un filtre à UV.

12. Composition de conditionnement selon l'une quelconque des revendications précédentes, **caractérisée en ce qu'**elle comprend au moins un agent antipelliculaire,

13. Utilisation des compositions selon l'une quelconques des revendications précédentes pour renforcer le brillant des cheveux.
